# EUROPÄISCHE PATENTSCHRIFT

(11) **EP 3 139 991 B1**
(45) Veröffentlichungstag und Bekanntmachung des Hinweises auf die Patenterteilung: **03.07.2019**
(21) Anmeldenummer: 15738241.7
(22) Anmeldetag: 30.04.2015
(51) Int. Cl.: A61M 39/16, A61M 39/00, A61M 39/10, A61M 39/02, A61M 5/165

(54) **VERBINDUNGSEINRICHTUNG FÜR EINEN KATHETER, INSBESONDERE EINEN PERIPHEREN VENENKATHETER**
CONNECTOR DEVICE FOR A CATHETER, PARTICULARLY A PERIPHERAL VENOUS CATHETER
MOYEN DE CONNEXION POUR UN CATHÉTER, EN PARTICULIER UN CATHÉTER VEINEUX PÉRIPHÉRIQUE

(30) Priorität: 08.05.2014 DE 102014106442
(43) Veröffentlichungstag der Anmeldung: 15.03.2017
(73) Patentinhaber: SICOS GmbH, 40229 Düsseldorf (DE)
(72) Erfinder: MARKUS, Kai, 52249 Eschweiler (DE); FRAUENRATH, Christian, 52428 Jülich (DE); REICHARDT, André, 99636 Rastenberg/Thürigen (DE)
(74) Vertreter: Naeven, Ralf
(86) Internationale Anmeldenummer: PCT/DE2015/100177
(87) Internationale Veröffentlichungsnummer: WO 2015/169282

(56) Entgegenhaltungen:
- EP-A1- 1 057 495
- EP-A1- 1 057 495
- EP-B1- 1 579 887
- WO-A1-95/21648
- WO-A1-2006/116955
- DE-A1-102012 108 791
- DE-A1-102012 108 791
- US-A- 5 049 139
- US-A- 5 049 139
- US-A- 5 190 534
- US-A- 5 190 534
- US-A- 5 782 808

## Beschreibung

Die Erfindung betrifft eine Verbindungseinrichtung für einen Katheter, insbesondere einen peripheren Venenkatheter, mit den Merkmalen des Oberbegriffs des Anspruchs 1.

Eine Verbindungseinrichtung der vorgenannten Art ist aus der EP 1 579 878 B1 bekannt, welche insbesondere bei zentralen Venenkathetern, die über lange Zeiträume am menschlichen Körper verbleiben, geeignet ist, die Gefahr von durch den Eintritt von Bakterien verursachten Sepsen zu verringern. Zentrale Venenkatheter können z. B. zur künstlichen Ernährung bei Kurzdarmsyndrom eingesetzt werden. Derartige Sepsen sind für die behandelte Person mit erheblichen persönlichen Nachteilen verbunden. Als Lösung schlägt der genannte Stand der Technik vor, ein zahlreiche Auslassöffnungen aufweisendes Siebelement aus Silber vorzusehen, welches im Infusionskanal oder in Infusionsflussrichtung gesehen am Ende des Infusionskanals angeordnet ist. Das als Siebelement ausgebildete Durchflusselement stellt eine Barriere für Bakterien und/oder Pilze dar und ist auch deshalb vorteilhaft, weil die komplette Verbindungseinrichtung zusammen mit dem Siebelement auf sehr einfache Art und Weise ausgetauscht werden kann, wenn das Siebelement z. B. in Folge einer Verstopfung unwirksam werden sollte.

Aus US 5 782 808 A ist eine Verbindungseinrichtung für einen Katheter bekannt, in dem ein Durchflusselement fixiert angeordnet ist.

Es ist nun Aufgabe der vorliegenden Erfindung, eine Verbindungseinrichtung der eingangs genannten Art zur Verfügung zu stellen, welche die Schutzwirkung gegenüber Sepsen weiter verbessern kann.

Diese Aufgabe wird bei einer Verbindungseinrichtung der eingangs genannten Art durch Merkmale des Anspruchs 1 gelöst. Beispielhafte und vorteilhafte Ausbildungsformen der Verbindungseinrichtung sind in den abhängigen Ansprüchen definiert.

Demnach ist zunächst vorgeschlagen, einen Anschlusskörper, welcher einen Anschlusskörper-Infusionskanal aufweist, lösbar am Hauptkörper der Verbindungseinrichtung zu fixieren und das Durchflusselement in Längsrichtung des Hauptkörper-Infusionskanals verschiebbar zu lagern, wobei sich das Durchflusselement zumindest auch im Anschlusskörper-Infusionskanal erstreckt.

Das Durchflusselement wirkt somit auch zumindest auf einer Teilstrecke im Anschlusskörper-Infusionskanal sterilisierend. Durch eine langgestreckte Sackform kann die sterilisierende Wirkung des Durchflusselements über eine entsprechend lange Wegstrecke gewährleistet werden. Mit der Lösbarkeit des Anschlusskörpers am Hauptkörper ist das Durchflusselement zur Säuberung oder zum Austausch leicht erreichbar.

Die schwimmende Lagerung des Durchflusselements, vorzugsweise im Hauptkörper, sorgt dafür, dass sich der Abstand zwischen der äußeren Wand des Durchflusselements und der Innenwand des Anschlusskörper-Infusionskanal in Abhängigkeit von der im Infusionskanal gegebenen Durchflussrate verändern kann. Bei geringen Durchflussraten kann der Abstand zwischen dem Durchflusselement und der Wand des Anschlusskörper-Infusionskanals gering bleiben. Weist der Anschlusskörper-Infusionskanal ebenfalls eine sterilisierende Wirkung auf, ist das Verhältnis der sterilisierenden Oberfläche zum Volumen der in Kontakt zur sterilisierenden Oberfläche befindlichen Flüssigkeit sehr groß, was einen erhöhten Schutz bedeutet. Muss die Durchflussrate erhöht werden, würde ein zu geringer Abstand zwischen Durchflusselement und Infusionskanalwand möglicherweise in nicht gewünschter Weise flusshemmend wirken. Die schwimmende Lagerung des Durchflusselements erlaubt nun eine sich automatisch ergebende Abstandvergrößerung und somit einen geringeren Flusswiderstand.

Hierfür kann es vorteilhaft sein, dass die Form der das Durchflusselement umgebenden Wandung des Infusionskanals zumindest streckenweise an die Form des Durchflusselements, insbesondere durch einen parallelen Verlauf, angepasst ist.

Das Durchflusselement ist sackförmig, wobei eine "Sackform" auch sich in Flussrichtung verjüngende Formen umfasst. Eine Verjüngung kann vorteilhaft sein, insbesondere eine in Flussrichtung konus- oder einer kegelstumpfförmige Verjüngung. Eine entsprechende Form könnte der Infusionskanal zumindest streckenweise in dem das Durchflusselement umgebenden Bereich aufweisen, vorzugsweise mit einem zumindest streckenweise parallelen Verlauf von Durchflusselement-Außenwand und Infusionskanal-Innenwand.

Das Durchflusselement weist vorzugsweise eine Sieböffnungen aufweisende Wandung auf, z.B. in einer Umfangswand und/oder einer einer Eingangsöffnung des Durchflusselements gegenüberliegenden Endwand, und kann z. B. aus Silber bestehen oder an der Oberfläche mit Silber beschichtet sein. Anstelle von Silber kann auch eine silberhaltige Legierung oder chemische Verbindung oder ein sonstiges sterilisierend wirkendes Material, eingesetzt werden, z.B. Kupfer oder z.B. ein Kunststoff mit bakteriziden, antibiotischen und/oder zytostatischen Additiven. Das Additiv, beispielsweise in Form von Einschlüssen oder einer Dotierung, kann beispielsweise Silber oder Kupfer sein.

Die erfindungsgemäße Verbindungseinrichtung kann auch so ausgebildet sein, dass eine Umfangswand des Durchflusselements flüssigkeitsdicht ist und das Durchflusselement in einer einer Eingangsöffnung gegenüberliegenden Endwand mindestens eine Auslassöffnung aufweist und das Durchflusselement bei Verbindung des Hauptkörpers mit dem Anschlusskörper mit der Wandung des Anschlusskörper-Infusionskanals eine Presspassung eingeht. In diesem Fall ist das Durchflusselement in der Umfangswand undurchlässig für eine Flüssigkeit, insbesondere für die Infusionsflüssigkeit, die damit allein durch die Endwand hindurch in den Anschlusskörper-Infusionskanal eintritt. Da die Presspassung des Endes des Durchflusselements mit der Innenwand des Anschlusskörper-Infusionskanals dichtend ist, wird hierdurch die Dichtigkeit der Verbindung zwischen dem Hauptkörper und dem Anschlusskörper gewährleistet. Mit der dichtenden Presspassung können weitere Dichtungsmaßnahmen unterstützt oder auch entbehrlich werden. Mit der Presspassung können zudem Fertigungstoleranzen bei den Abmessungen des Anschlusskörper-Infusionskanals zu einem gewissen Maß aufgefangen werden. Die verschiebbare Lagerung des Durchflusselements ist in diesem Fall ebenfalls flüssigkeitsdicht zu gestalten.

Ist die Lagerung des Durchflusselements im Hauptkörper vorgesehen, ist im Falle der Presspassung zwischen Durchflusselement und Innenwand des Anschlusskörper-Infusionskanals die Verschiebbarkeit der Lagerung in Längsrichtung des Infusionskanals vorteilhaft, da auf diese Weise Toleranzen bei der Fertigung des Anschlusskörpers aufgefangen werden können. Mit der verschiebbaren Lagerung ist es auch möglich, die Presspassung bei unterschiedlichen Bauformen oder Abmessungen des Anschlusskörpers zu realisieren.

Zum Erreichen der Presspassung weisen das Durchflusselement und die Innenwand des Anschlusskörper-Infusionskanals geeignete Formen auf. Beispielsweise kann sich die Innenwand des Anschlusskörper-Infusionskanals in Flussrichtung der Infusionsflüssigkeit verjüngen, beispielsweise konisch mit einem bestimmten Öffnungswinkel geformt sein. In diesem Fall könnte das Durchflusselement eine zylindrisch geformte Außenwand aufweisen oder sich ebenfalls in Flussrichtung verjüngen, wobei die pro Längeneinheit gegebene Durchmesserverringerung der Außenseite der Umfangswand des Durchflusselements geringer ist, als die pro Längeneinheit gegebene Durchmesserverringerung der Innenwand des Anschlusskörper-Infusionskanals. Im Falle einer Konusform weist die Außenseite der Umfangswand des Durchflusselements einen geringeren Öffnungswinkel als die Innenwand des Anschlusskörper-Infusionskanals auf.

Es kann in der Endwand eine Vielzahl von Auslassöffnungen vorgesehen sein, so dass eine siebartige Struktur entsteht. Alternativ oder zusätzlich kann (können) auch eine oder mehrere größere Auslassöffnung(en) in der Endwand vorgesehen sein.

Die erfindungsgemäße Verbindungseinrichtung kann auch so ausgebildet sein, dass im Inneren des Durchflusselements mindestens ein sterilisierend wirkender Einsatzkörper angeordnet ist. Der mindestens eine Einsatzkörper vergrößert die sterilisierende Oberfläche und kann unabhängig von der Anzahl der Auslassöffnungen eingesetzt werden. Der mindestens eine Einsatzkörper kann zum Beispiel in Durchflussrichtung gesehen einen kreuzförmigen Querschnitt aufweisen. Der mindestens eine Einsatzkörper im Inneren des Durchflusselements kann gesondert und auswechselbar oder einstückig mit dem Durchflusselement sein. Der mindestens eine Einsatzkörper kann aus demselben Material wie der Hauptkörper bestehen. Der Einsatzkörper oder mindestens einer der Einsatzkörper kann aber auch aus einem anderen Material als dem des Hauptkörpers bestehen. Als Material des mindestens einen Einsatzkörpers kommt z.B. massives Silber oder Kupfer oder eine massive Silber und/oder Kupfer enthaltende Legierung oder ein sterilisierend dotierter Kunststoff in Frage, wobei im Falle mehrerer Einsatzkörper diese auch aus unterschiedlichen Materialien bestehen können.

Für eine zusätzliche sterilisierende Wirkung der Verbindungseinrichtung kann vorgesehen werden, dass auch der Hauptkörper zumindest im Hauptkörper-Infusionskanal sterilisierend wirkt, z.B. durch Verwendung eines sterilisierend wirkenden Massivmaterials oder einer sterilisierend wirkenden Beschichtung oder Dotierung des Hauptkörpers. Unter einem Massivmaterial wird ein Material verstanden, welches ohne Auftrag auf einen Träger oder ein tragendes Substrat eine Formstabilität aufweist. Folien oder auf ein Substrat aufgetragene dünne Schichten sind somit kein Massivmaterial. Ein Massivmaterial kann jedoch eine mit Fremdstoffen dotierte oder Fremdelemente, z.B. Verstärkungselemente, insbesondere Fasern, enthaltende Matrix umfassen. Das Matrixmaterial selbst und/oder die Fremdstoffe oder Fremdelemente können eine sterilisierende Wirkung aufweisen.

Die erfindungsgemäße Verbindungseinrichtung kann auch so ausgebildet sein, dass der Anschlusskörper an seinem dem Hauptkörper abgewandten Ende ein Koppelstück für den Anschluss an eine Kanüle, insbesondere an eine Flügelkanüle aufweist. Hierdurch kann das Anlegen eines Katheters beschleunigt und vereinfacht werden. Zwischenelemente, z.B. in Schlauchform, welche zudem kontaminiert sein können und deren Anschluss Zeit kostet, werden hierdurch verzichtbar.

Es kann aber auch vorteilhaft sein, die erfindungsgemäße Verbindungseinrichtung so auszugestalten, dass der Anschlusskörper Teil einer Kanüle, insbesondere einer Flügelkanüle ist.

Es kann auch vorteilhaft sein, zwischen Hauptkörper-Infusionskanal und Anschlusskörper-Infusionskanal eine dichtende Presspassung vorzusehen. Hierdurch ist eine (zusätzliche) Sicherung gegen den Austritt von Infusionsflüssigkeit nach Außen oder den ungewollten Eintritt von Substanzen gegeben.

Im Folgenden werden bevorzugte Ausführungsformen der erfindungsgemäßen Verbindungseinrichtung anhand von Figuren vorgestellt.

Es zeigt:
- Fig. 1:: eine erste Ausführungsform einer Verbindungseinrichtung mit Sieblöchern in der Umfangswand einer ersten Variante eines Durchflusselements,
- Fig. 2:: eine zweite Ausführungsform einer Verbindungseinrichtung mit Presspassung zwischen einer zweiten Variante eines Durchflusselements und Infusionskanal eines Anschlusskörpers,
- Fig. 3:: die zweite Variante des Durchflusselements mit einem siebartigen Boden in perspektivischer Ansicht,
- Fig. 4:: eine dritte Variante des Durchflusselements mit sterilisierendem Einsatzkörper in perspektivischer Ansicht und
- Fig. 5:: das Durchflusselement nach Fig. 4 in Aufsicht.

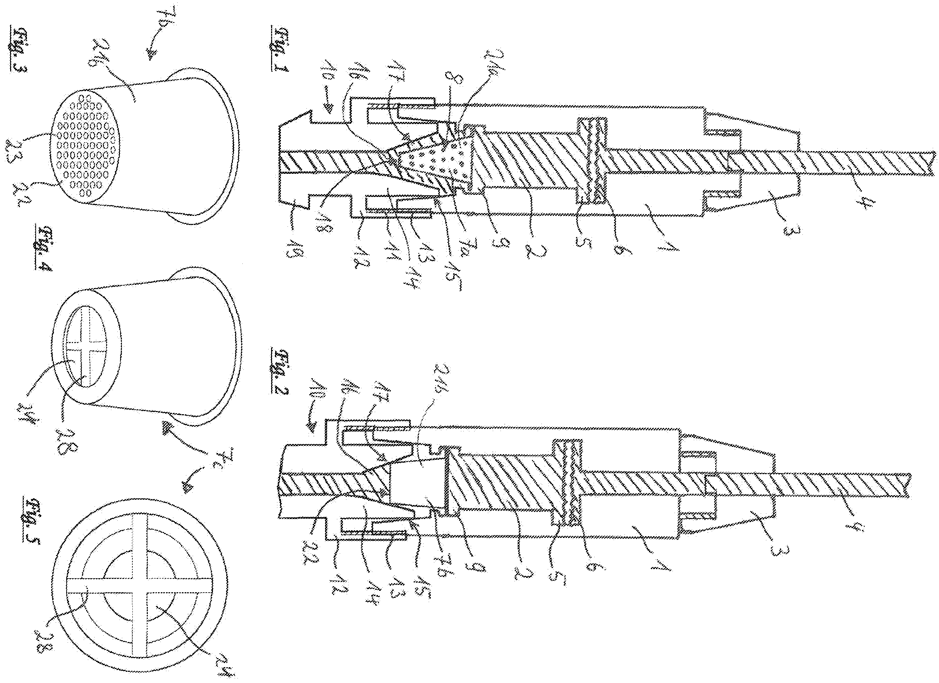

In schematischer Darstellung zeigt Fig. 1 eine erste Verbindungseinrichtung mit einem Hauptkörper 1, durch den ein Hauptkörper-Infusionskanal 2 verläuft. Der Hauptkörper 1 ist über ein Verbindungselement 3 an einen Infusionsschlauch 4 gekoppelt. Im Hauptkörper-Infusionskanal 2 ist eine Aufweitung 5 vorgesehen, welche einen Bakterienfilter 6 aufweist.

Am unteren Ende des Hauptkörper-Infusionskanals 2 ist ein siebartiges Durchflusselement 7a vorgesehen, welches in seiner Umfangswand 21a zahlreiche Sieböffnungen 8 aufweist. Das Durchflusselement 7a besteht aus einem sterilisierenden Material und/oder ist mit einem sterilisierenden Material beschichtet. Das sterilisierende Material kann Silber sein. Das Durchflusselement 7a ist in einem im Hauptkörper-Infusionskanal 2 vorgesehenen Rücksprung 9 schwimmend derart gelagert, dass das Durchflusselement 7a in Längsrichtung des Hauptkörpers 1 verschiebbar ist.

Ein Anschlusskörper 10 ist mittels eines ein Innengewinde 11 aufweisenden Mutterelementes 12 an einem Außengewinde 13 des Hauptkörpers 1 fixiert. Der Anschlusskörper 10 weist einen Eingriffsstutzen 14 auf, der in einen Endbereich 15 des Hauptkörper-Infusionskanals 2 eingreift. Der Eingriffsstutzen 14 ist aus einem flexiblen Material und relativ zum Endbereich 15 des Hauptkörpers 1 derart dimensioniert, dass es bei der Fixierung des Anschlusskörpers 10 zu einer Presspassung zwischen dem Eingriffsstutzen 14 und dem Endbereich 15 des Hauptkörpers 1 kommt.

Bei fixiertem Anschlusskörper 10 ragt das Durchflusselement 7a in einen Anschlusskörper-Infusionskanal 16 hinein. In einem Eingangsbereich 17 ist der Anschlusskörper-Infusionskanal 16 aufgeweitet, wobei die Innenwand des Eingangsbereichs 17 im Wesentlichen parallel zur Außenwand des konisch geformten Durchflusselements 7a ist.

Das Durchflusselement 7a weist an seinem Ende eine senkrecht zur Längsrichtung des Hauptkörper-Infusionskanals 2 orientierte Endwand 18 auf, welche ebenfalls feine Auslassöffnungen, d.h. Sieböffnungen 8, aufweist. Die Endwand 18 kann-wie dargestellt - flach sein oder eine beliebige andere, z.B. auch bauchige, Form aufweisen. Damit ist gewährleistet, dass auch bei einem Aufliegen der Umfangswand des Durchflusselements 7a auf der Innenwand des Anschlusskörper-Infusionskanals 16 im Eingangsbereich 17, welches ein Verschließen der Sieblöcher 8 der Endwand 18 zur Folge haben kann, ein Durchfluss gegeben ist.

Der Anschlusskörper 10 weist an seinem Ende ein Koppelstück 19 für den Anschluss an eine hier nicht dargestellte Kanüle, insbesondere an eine Flügelkanüle, auf. Das Koppelstück 19 ist hier lediglich schematisch angedeutet und kann eine völlig andere Gestalt und Kopplungsmechanik aufweisen, z.B. Teil eines Luer-Locks sein.

Fig. 2 zeigt eine weitere Variante einer Verbindungseinrichtung, die in vielen Merkmalen mit der Verbindungseinrichtung gemäß Fig. 1 übereinstimmt. Übereinstimmende Elemente sind daher mit denselben Bezugszeichen versehen. Es wird diesbezüglich auf die Beschreibung zu Fig. 1 verwiesen. Anders als in Fig. 1 ist in der Ausführungsform gemäß Figur 2 eine zweite Variante eines Durchflusselements 7b vorgesehen, welches in Fig. 3 vergrößert in einer perspektivischen Ansicht von schräg unten dargestellt ist. Die Umfangswand 21b des Durchflusselements 7b ist konisch geformt, weist aber einen deutlich geringeren Öffnungswinkel auf als die Innenwand des Eingriffsstutzens 14. Mit der Verbindung zwischen Hauptkörper 1 und Anschlusskörper 10 kommt es zu einer Presspassung zwischen dem Durchflusselement 7b und dem Eingriffsstutzen 14. Die Umfangswand 21 des Durchflusselements 7b weist keine Sieböffnungen auf. Eine Endwand 22 weist hingegen eine Vielzahl von Sieböffnungen 23 auf. Soweit die Lagerung des Durchflusselements 7b im Rücksprung 9 des Hauptkörpers 1 dichtend gegenüber der Infusionsflüssigkeit ausgestaltet ist, weist das Durchflusselement 7b insgesamt eine Dichtfunktion auf, da auch die Presspassung zwischen Durchflusselement 7b und Eingriffsstutzen 14 dichtend ist. Eine Dichtfunktion in der Verbindung zwischen Zwischenstück 10 und Hauptkörper 1 ist somit nicht mehr erforderlich oder wird zumindest durch diese Ausbildung des Durchflusselements 7b unterstützt.

Das Durchflusselement 7b kann in seiner Umfangswand 21 selbstverständlich auch zylindrisch geformt sein.

Die Fig. 4 und 5 zeigen eine alternative Ausführungsform eines Durchflusselements 7c, welches anstelle des Durchflusselements 7a oder 7b eingesetzt werden kann. Das Durchflusselement 7c weist anstelle von Sieböffnungen lediglich eine einzige Auslassöffnung 24 auf, wobei im Durchflusselement 7c ein sterilisierend wirkender kreuzförmiger Einsatzkörper 28 vorgesehen ist.

### Bezugszeichenliste

- 1.: Hauptkörper
- 2.: Hauptkörper-Infusionskanal
- 3.: Verbindungselement
- 4.: Infusionsschlauch
- 5.: Aufweitung
- 6.: Bakterienfilter
- 7.: Durchflusselement
- 8.: Sieböffnung
- 9.: Rücksprung
- 10.: Anschlusskörper
- 11.: Innengewinde
- 12.: Mutterelement
- 13.: Außengewinde
- 14.: Eingriffsstutzen
- 15.: Endbereich
- 16.: Anschlusskörper-Infusionskanal
- 17.: Eingangsbereich
- 18.: Endwand
- 19.: Koppelstück
- 20.: Durchflusselement
- 21.: Umfangswand
- 22.: Endwand
- 23.: Sieböffnungen
- 24.: Auslassöffnung
- 28.: Einsatzkörper

## Patentansprüche

1. Verbindungseinrichtung für einen Katheter, insbesondere einen peripheren Venenkatheter, umfassend
einen Hauptkörper (1) mit einem Hauptkörper-Infusionskanal (2) und
ein sterilisierend wirkendes und sich sackförmig in Flussrichtung des Hauptkörper-Infusionskanals (2) erstreckendes Durchflusselement (7a, 7b, 7c), wobei
ein einen Anschlusskörper-Infusionskanal (16) aufweisender Anschlusskörper (10) lösbar am Hauptkörper (1) fixiert ist und
das Durchflusselement (7a, 7b, 7c) sich zumindest auch im Anschlusskörper-Infusionskanal (16) erstreckt,
**dadurch gekennzeichnet, dass**
das Durchflusselement (7a, 7b, 7c) bei am Hauptkörper (1) konnektiertem Anschlusskörper (10) in Längsrichtung des Hauptkörper-Infusionskanals (2) verschiebbar bezüglich des Anschlusskörpers (10) gelagert ist.

2. Verbindungseinrichtung nach Anspruch 1, **dadurch gekennzeichnet, dass** das Durchflusselement (7a, 7b, 7c) am Hauptkörper (1) gelagert ist.

3. Verbindungseinrichtung nach Anspruch 1 oder 2, **dadurch gekennzeichnet, dass** sich der Anschlusskörper-Infusionskanal (16) zumindest auf einem das Durchflusselement (7a, 7b, 7c) umgebenden Streckenstück (17) in Flussrichtung verjüngt.

4. Verbindungseinrichtung nach einem der vorhergehenden Ansprüche, **dadurch gekennzeichnet, dass** die Form der das Durchflusselement (7a, 7b, 7c) umgebenden Wandung des Hauptkörper-Infusionskanals (2) und/oder des Anschlusskörper-Infusionskanals (16) zumindest streckenweise an die Form des Durchflusselements (7a, 7b, 7c), insbesondere durch einen parallelen Verlauf, angepasst ist.

5. Verbindungseinrichtung nach einem der vorhergehenden Ansprüche, **dadurch gekennzeichnet, dass** der Anschlusskörper (10) zumindest auf einer Teilstrecke im Anschlusskörper-Infusionskanal (16) eine sterilisierende Wirkung aufweist.

6. Verbindungseinrichtung nach einem der vorhergehenden Ansprüche, **dadurch gekennzeichnet, dass** zwischen Hauptkörper-Infusionskanal (2) und Anschlusskörper-Infusionskanal (16) eine dichtende Presspassung vorgesehen ist.

7. Verbindungseinrichtung nach einem der vorhergehenden Ansprüche, **dadurch gekennzeichnet, dass** im Inneren des Durchflusselements (7a, 7b, 7c) mindestens ein sterilisierend wirkender Einsatzkörper (28) angeordnet ist.

8. Verbindungseinrichtung nach einem der vorhergehenden Ansprüche, **dadurch gekennzeichnet, dass** der Anschlusskörper (10) an seinem dem Hauptkörper (1) abgewandten Ende ein Koppelstück (19) für den Anschluss an eine Kanüle, insbesondere an eine Flügelkanüle, aufweist.

9. Verbindungseinrichtung nach einem der Ansprüche 1 bis 7, **dadurch gekennzeichnet, dass** der Anschlusskörper (10) Teil einer Kanüle, insbesondere einer Flügelkanüle, ist.

## Claims

1. Connection device for a catheter, especially for a peripheral venous catheter, comprising
a main body (1) with a main body infusion channel (2) and a through-flow element (7a, 7b, 7c) having sterilizing effect and extending in the form of a bag in flow direction of the main body infusion channel (2), wherein
an attachment body (10) having an attachment body infusion channel (16) is detachably fixed to the main body (1) and
the through-flow element (7a, 7b, 7c) extends at least also into the attachment body infusion channel (16),
**characterized in that**
the through-flow element (7a, 7b, 7c) is mounted displaceably in the longitudinal direction of the main body infusion channel (2) with respect to the attachment body (10) when the attachment body (10) is connected to the main body (1).

2. Connection device according to claim 1, **characterized in that** the through-flow element (7a, 7b, 7c) is mounted on the main body (1).

3. Connection device according claim 1 or 2, **characterized in that** the attachment body infusion channel (16) tapers in the direction of flow at least in a section (17) surrounding the through-flow element (7a, 7b, 7c).

4. Connection device according to one of the preceding claims, **characterized in that** the shape of the wall of the main body infusion channel (2), said wall surrounding the through-flow element (7a, 7b, 7c), and/or the shape of the attachment body infusion channel (16 is aligned, at least in part, to the shape of the through-flow element (7a, 7b, 7c), in particular by a parallel course.

5. Connection device according to one of the preceding claims, **characterized in that** the attachment body (10) has a sterilizing effect in the attachment body infusion channel (16), at least on a section.

6. Connection device according to one of the preceding claims, **characterized in that** between main body infusion channel (2) and attachment body infusion channel (16) a sealing press fit is provided.

7. Connection device according to one of the preceding claims, **characterized in that** at least one sterilizing insert body (28) is arranged inside the through-flow element (7a, 7b, 7c).

8. Connection device according to one of the preceding claims, **characterized in that** the attachment body (10) has, at its end facing away from the main body (1), a coupling piece (19) for connection to a cannula, in particular a butterfly cannula.

9. Connection device according to one of claims 1 to 7, **characterized in that** the attachment body (10) is part of a cannula, in particular of a wing cannula.

## Revendications

1. Dispositif de connexion pour cathéter, en particulier un cathéter veineux périphérique, comprenant
un corps principal (1) avec un canal de perfusion du corps principal (2) et un élément d'écoulement (7a, 7b, 7c) ayant un effet stérilisant et s'étendant en forme de sac dans la direction d'écoulement du canal de perfusion du corps principal (2), dans lequel un corps de connexion (10) ayant un canal de perfusion du corps de connexion (16) est fixé de manière amovible sur le corps principal (1) et l'élément d'écoulement (7a, 7b, 7c) s'étend au moins également dans le canal de perfusion du corps de connexion (16), **caractérisé en ce que**
l'élément d'écoulement (7a, 7b, 7c) est monté coulissant par rapport au corps de connexion (10) dans la direction longitudinale du canal de perfusion du corps principal (2), quand le corps de connexion (10) est connecté au corps principal (1).

2. Dispositif de connexion selon la revendication 1, **caractérisé en ce que** l'élément d'écoulement (7a, 7b, 7c) est monté sur le corps principal (1).

3. Dispositif de connexion selon la revendication 1 ou 2, **caractérisé en ce que** le canal de perfusion du corps de connexion (16) se rétrécit dans la direction d'écoulement au moins sur une section (17) entourant l'élément d'écoulement (7a, 7b, 7c).

4. Dispositif de connexion selon l'une des revendications précédentes, **caractérisé en ce que** la forme de la paroi du canal de perfusion du corps principal (2) entourant l'élément d'écoulement (7a, 7b, 7c) et / ou la forme du canal de perfusion du corps de connexion (16) est adaptée au moins en partie à la forme de l'élément d'écoulement (7a, 7b, 7c), en particulier par une trajectoire parallèle.

5. Dispositif de connexion selon l'une des revendications précédentes, **caractérisé en ce que** le corps de connexion (10) a un effet stérilisant au moins sur un trajet partiel dans le canal de perfusion du corps de connexion (16).

6. Dispositif de connexion selon l'une des revendications précédentes, **caractérisé en ce qu'**un ajustement forcé de manière étanche est prévu entre le canal de perfusion du corps principal (2) et le canal de perfusion du corps de connexion (16).

7. Dispositif de connexion selon l'une des revendications précédentes, **caractérisé en ce qu'**au moins un corps d'insertion stérilisant (28) est disposé à l'intérieur de l'élément d'écoulement (7a, 7b, 7c).

8. Dispositif de connexion selon l'une des revendications précédentes, **caractérisé en ce que** le corps de connexion (10) présente à son extrémité opposée au corps principal (1) une pièce d'accouplement (19) destinée à être connectée à une canule, en particulier à une canule papillon.

9. Dispositif de connexion selon l'une des revendications 1 à 7, **caractérisé en ce que** le corps de connexion (10) fait partie d'une canule, en particulier d'une canule papillon.
